# EUROPÄISCHE PATENTANMELDUNG

(11) **EP 0 701 115 A1**
(43) Veröffentlichungstag der Anmeldung: **13.03.1996**
(21) Anmeldenummer: 95113957.5
(22) Anmeldetag: 06.09.1995
(51) Int. Cl.: G01N 1/22, G01N 21/78, G01N 33/00

(54) **Detektionsvorrichtung mit einem Gasspürelement**

(30) Priorität: 10.09.1994 DE 4432218
(71) Anmelder: DRÄGERWERK AKTIENGESELLSCHAFT, D-23542 Lübeck (DE)
(72) Erfinder: Formica, Philip Micheal, D-23558 Lübeck (DE); May, Wolfgang, Dr., D-23858 Reinfeld (DE); Evers, Lore, D-23564 Lübeck (DE)

(57) **Zusammenfassung**

Eine Detektionsvorrichtung mit einem Gasspürelement (4, 5, 6) zur Untersuchung von in einem Reservoir (11) befindlichen Druckgasen durch Probenahme unter Druckminderung, mit einer Kupplungsvorrichtung (8, 12) zum Verbinden der Detektionsvorrichtung mit einem Reservoir (11) soll hinsichtlich der Handhabbarkeit im Routinebetrieb verbessert werden. Zur Lösung der Aufgabe ist vorgesehen, daß die Detektionsvorrichtung ein die Dauer des auf einen vorgewählten Wert eingestellten Probenahme-Flusses erfassendes Zeitmeßgerät (22) mit einem das Zeitmeßgerät (22) startenden Schalter (9) aufweist, und daß der Schalter (9) zumindestens beim Ankuppeln der Detektionsvorrichtung an das Reservoir (11) betätigt ist.

## Beschreibung

Die Erfindung betrifft eine Detektionsvorrichtung mit einem Gasspürelement zur Untersuchung von in einem Reservoir befindlichen Druckgasen durch Probenahme unter Druckminderung und mit einer Kupplungsvorrichtung zum Verbinden der Detektionsvorrichtung mit dem Reservoir.

Eine Detektionsvorrichtung der genannten Art ist aus der DE-C 27 31 361 bekanntgeworden. Die Detektionsvorrichtung ist über eine Kupplung an einen Druckgasbehälter oder eine Druckgasleitung angeschlossen. Sie beinhaltet neben der Kupplung einen Druckminderer, einen den Probenahmefluß unterbrechenden Absperrhahn, einen Durchflußmengenmesser zur Messung des Probenahmeflusses und ein dem Durchflußmengenmesser nachgeschaltetes Gasprüfröhrchen. Zur Durchführung einer Gasanalyse wird ein vorbestimmtes Probegasvolumen durch das Gasprüfröhrchen geleitet, und es wird anhand der Verfärbung der Reaktionsschicht des Gasprüfröhrchens der Anteil der nachzuweisenden Komponente im Druckgas bestimmt.

Nachteilig bei der bekannten Vorrichtung ist, daß zur Einstellung des Probegasvolumens ein separates Zeitmeßgerät erforderlich ist, welches im täglichen Routinebetrieb häufig nicht in Griffnähe liegt. Da vom Benutzer außerdem das Intervall der Probegasnahme mit dem Zeitmeßgerät manuell erfasst werden muß, sind Abweichungen des einzustellenden Probegasvolumens vom tatsächlich vorliegenden Probegasvolumen nicht auszuschließen, und der am Gasprüfröhrchen abgelesene Konzentrationswert ist dann ungenau.

Aus der DE-PS 10 93 113 ist eine Vorrichtung zur Messung bestimmter Bestandteile in Luft oder anderen Gasen bekanntgeworden, bei welcher mittels Steuerung durch eine Schaltuhr einzelne Prüfröhrchen in bestimmten Zeitintervallen einem vorbestimmten Probegasvolumen ausgesetzt sind, indem sie durch ein von der Schaltuhr gesteuertes Zahnstangengetriebe an den Probegasstrom angeschlossen werden. Die bekannte Vorrichtung ist für die Langzeitüberwachung von Gaskonzentrationen, beispielsweise in Arbeitsräumen, konzipiert, ohne daß eine Bedienungsperson an der Messung beteiligt ist.

Der Erfindung liegt die Aufgabe zugrunde, eine Detektionsvorrichtung der genannten Art hinsichtlich ihrer Handhabbarkeit im Routinebetrieb zu verbessern.

Zur Lösung der Aufgabe ist vorgesehen, daß die Detektionsvorrichtung ein die Dauer des auf einen vorgewählten Wert eingestellten Probenahme-Flusses erfassendes Zeitmeßgerät mit einem das Zeitmeßgerät startenden und/oder stoppenden Schalter aufweist und daß der Schalter zumindestens beim Ankuppeln der Detektionsvorrichtung an das Reservoir betätigt ist.

Der Vorteil der Erfindung besteht im wesentlichen darin, daß die Detektionsvorrichtung mit einem Zeitmeßgerät versehen ist, welches beim Verbinden der Detektionsvorrichtung mit dem Reservoir aktiviert wird. Der Startpunkt der Zeitmessung verläuft damit synchron zum Beginn der Probegasnahme. Das Zeitmeßgerät besteht aus einer Uhr und einer Steuerlogik, wobei die Steuerlogik mit dem Schalter verbunden ist und je nach Schaltposition des Schalters die Uhr startet oder stoppt. Durch Einstellung des Probenahme-Flusses auf einen vorgewählten Wert und Erfassung der Probenahme-Zeit durch das Zeitmeßgerät kann der Meßablauf weitestgehend automatisiert werden. Der die Uhr im Zeitmeßgerät aktivierende Schalter wird zumindestens beim Ankuppeln der Detektionsvorrichtung an das Reservoir betätigt. Zweckmäßigerweise wird die Uhr beim Abkuppeln der Detektionsvorrichtung vom Reservoir selbsttätig mittels des Schalters gestoppt.

Vorteilhafte Ausgestaltungen der Erfindung sind in den Unteransprüchen angegeben.

In zweckmäßiger Weise ist der Schalter im Eingriffsbereich der aus einem Kupplungsstecker und einer Kupplungsmuffe bestehenden Kupplungsvorrichtung angeordnet.

In vorteilhafter Weise wird ein zwischen der Kupplungsvorrichtung und dem Gasspürelement befindliches Absperrelement durch den Schalter bzw. das Zeitmeßgerät betätigt. Auf diese Weise wird der Probenahmefluß nur bei aktiviertem Zeitmeßgerät freigegeben.

In zweckmäßiger Weise wird das Absperrelement nach Ablauf einer vorgewählten Meßzeit durch den Schalter und/oder das Zeitmeßgerät in Schließstellung geschaltet. So kann beispielsweise in die Steuerlogik eine feste Meßzeit einprogrammiert sein, nach deren Ablauf das Absperrventil geschlossen und die Uhr gleichzeitig angehalten wird.

In zweckmäßiger Weise sind als Gasspürelemente mehrere, strömungsmäßig parallel geschaltete Gasprüfröhrchen vorgesehen.

Ein Ausführungsbeispiel der Erfindung ist in der Zeichnung dargestellt und im folgenden näher erläutert:

Es zeigen:
- Figur 1: eine Aufsicht auf eine Detektionsvorrichtung,
- Figur 2: eine Seitenansicht der Detektionsvorrichtung nach der Figur 1,
- Figur 3: einen pneumatischen Schaltplan der Detektionsvorrichtung.

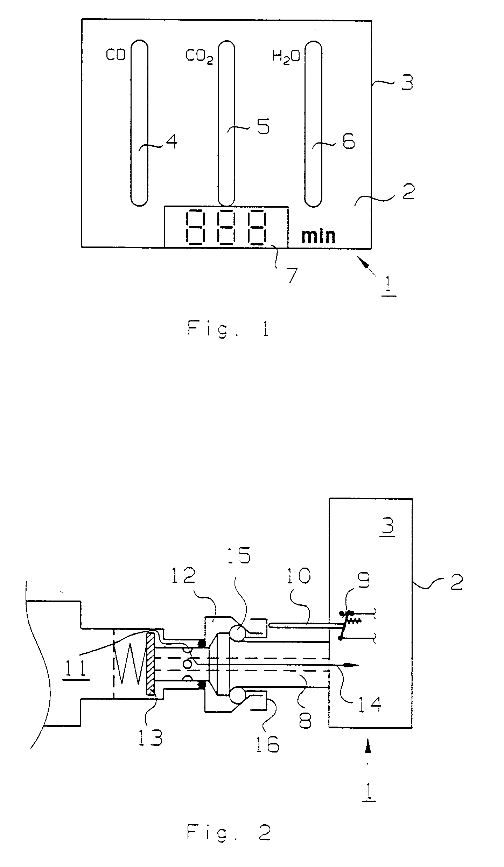

Bei der in Figur 1 gezeigten Detektionsvorrichtung (1) sind hinter einer Frontplatte (2) eines Gehäuses (3) ein erstes Gasprüfröhrchen (4) zum Nachweis von CO, ein zweites Gasprüfröhrchen (5) zum Nachweis von CO₂ und ein drittes Gasprüfröhrchen (6) zum Nachweis von H₂O-Dampf und eine Anzeige (7) eines in der Figur 1 nicht dargestellten Zeitmeßgerätes angeordnet.

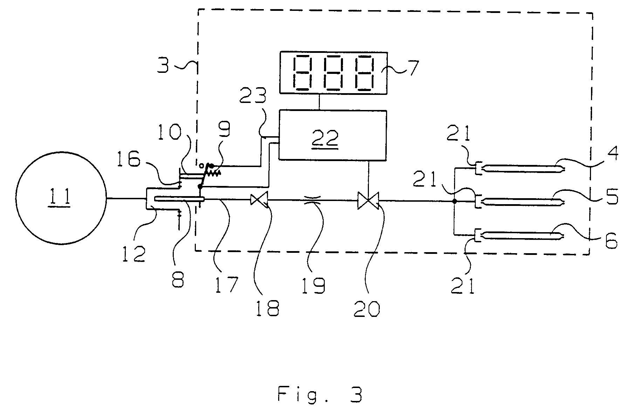

An der der Frontplatte (2) gegenüberliegenden Seite des Gehäuses (3) sind, wie in der Figur 2 veranschaulicht, ein Kupplungsstecker (8), und ein Schalter (9) mit einem Betätigungsstift (10) befestigt, wobei der Schalter (9) über den Betätigungsstift (10) im Eingriff mit einer Kupplungsmuffe (12) steht, in welche der Kupplungsstecker (8) eingesteckt ist. Die Kupplungsmuffe (12) ist mit einem mit Druckgas gefüllten Reservoir (11) verbunden, welches in der Figur 2 nur stückweise dargestellt ist. Innerhalb der Kupplungsmuffe (12) befindet sich ein Rückschlagventil (13), das bei herausgezogenem Kupplungsstecker (8) den Gasstrom aus dem Reservoir (11) unterbricht. Der Gasfluß vom Reservoir (11) in die Detektionsvorrichtung (1) ist durch einen Pfeil (14) veranschaulicht. Der Kupplungsstecker (8) ist innerhalb der Kupplungsmuffe (12) mit einer lösbaren Verriegelungsvorrichtung (15) arretiert, die durch Druck auf einen Ring (16) den Kupplungsstecker (8) freigibt.

Figur 3 zeigt schematisch den pneumatischen Schaltplan der Detektionsvorrichtung (1). Gleiche Komponenten sind mit gleichen Bezugsziffern der Figuren 1 und 2 bezeichnet. In der vom Kupplungsstecker (8) abgehenden Leitung (17) sind ein Druckminderer (18), eine Festdrossel (19), zur Einstellung eines vorgewählten Probenahme-Flusses, ein Absperrventil (20) und Analysenanschlüsse (21) zum Anschluß der Gasprüfröhrchen (4, 5, 6) angeordnet. Ein Zeitmeßgerät (22) ist mit Anschlußleitungen (23) des Schalters (9), mit dem Absperrventil (20) und mit der Anzeige (7) verbunden. Die Arbeitsweise der erfindungsgemäßen Detektionsvorrichtung (1) ist folgendermaßen: Beim Einstecken des Kupplungsstecker (8) in die Kupplungsmuffe (12) berührt der Betätigungsstift (10) den Ring (16) und der Schalter (9) schaltet in Schließstellung. Innerhalb des Zeitmeßgerätes (22) befindet sich eine in der Figur 3 nicht dargestellte Steuerlogik, welche bei geschlossenem Schalter (9) das Absperrventil (20) öffnet und die Uhr des Zeitmeßgerätes startet. Die Meßzeit kann in Minuten an der Anzeige (7) abgelesen werden. Bei geöffnetem Absperrventil (20) fließt der durch die Festdrossel (19) begrenzte Probenahmegasfluß durch die Gasprüfröhrchen (4, 5, 6). Nach Ablauf der Meßzeit, die normalerweise einige Minuten beträgt, wird der Kupplungsstecker (8) aus der Kupplungsmuffe (12) herausgezogen, der Betätigungsstift (10) kommt hierbei aus dem Eingriff mit dem Ring (16) und der Schalter (9) schaltet in Öffnungsstellung. Durch die Steuerlogik des Zeitmeßgerätes (22) wird die Uhr angehalten und das Absperrventil (20) geschlossen. An den Gasprüfröhrchen (4, 5, 6) können anhand der Verfärbungen ihrer Reaktionszonen die Konzentrationsanteile der nachzuweisenden Komponenten in der Gasprobe abgelesen werden.

## Patentansprüche

1. Detektionsvorrichtung mit einem Gasspürelement (4, 5, 6) zur Untersuchung von in einem Reservoir (11) befindlichen Druckgasen durch Probenahme unter Druckminderung, mit einer Kupplungsvorrichtung (8, 12) zum Verbinden der Detektionsvorrichtung (1) mit dem Reservoir (11) dadurch gekennzeichnet, daß die Detektionsvorrichtung (1) ein die Dauer des auf einen vorgewählten Wert eingestellten Probenahme-Flusses erfassendes Zeitmeßgerät (22) mit einem das Zeitmeßgerät (22) startenden und/oder stoppenden Schalter (9) aufweist, und daß der Schalter (9) zumindestens beim Ankuppeln der Detektionsvorrichtung (1) an das Reservoir (11) betätigt ist.

2. Detektionsvorrichtung nach Anspruch 1, dadurch gekennzeichnet, daß der Schalter (9) im Eingriffsbereich der aus einem Kupplungsstecker (8) und einer Kupplungsmuffe (12) bestehenden Kupplungsvorrichtung angeordnet ist.

3. Detektionsvorrichtung nach Anspruch 1 oder 2, dadurch gekennzeichnet, daß ein im Leitungszug zwischen der Kupplungsvorrichtung (8, 12) und dem Gasspürelement (4, 5, 6) befindliches Absperrelement (20) durch das Zeitmeßgerät (22) und/oder den Schalter (9) betätigt ist.

4. Detektionsvorrichtung nach Anspruch 3, dadurch gekennzeichnet, daß das Absperrelement (20) zumindestens nach Ablauf einer vorgewählten Meßzeit durch den Schalter (9) und/oder das Zeitmeßgerat (22) in Schließstellung geschaltet ist.

5. Detektionsvorrichtung nach einem der Ansprüche 1 bis 4, dadurch gekennzeichnet, daß als Gasspürelement mehrere, parallel begaste Gasprüfröhrchen (4, 5, 6) vorgesehen sind.
